# EUROPEAN PATENT SPECIFICATION

(11) **EP 3 370 700 B1**
(45) Date of publication and mention of the grant of the patent: **10.03.2021**
(21) Application number: 16812850.2
(22) Date of filing: 02.11.2016
(51) Int. Cl.: A61K 31/198, A61K 31/722, A61K 31/732, A61K 31/205, A61K 9/06, A61K 9/00, A61K 47/12, A61P 1/04

(54) **ORAL GEL COMPOSITION COMPRISING CHITOSAN, PECTIN, L-CARNITINE AND N-ACETYLCYSTEINE**
ORALE ZUSAMMENSETZUNG IN GELFORM MIT CHITOSAN, PEKTIN MIT HOHEM METHOXYLIERUNGSGRAD, EINER ZUCKERHALTIGEN SUBSTANZ, L-CARNITIN UND N-ACETYLCYSTEIN
COMPOSITION ORALE SOUS FORME DE GEL COMPRENANT DU CHITOSANE, UNE PECTINE À DEGRÉ ÉLEVÉ DE MÉTHOXYLATION, UNE SUBSTANCE SUCRÉE, LA L-CARNITINE ET LA N-ACÉTYLCYSTÉINE

(30) Priority: 03.11.2015 IT UB20154846
(43) Date of publication of application: 12.09.2018
(73) Proprietor: Labomar S.p.A., 31036 Istrana (TV) (IT)
(72) Inventor: FRATTER, Andrea, 30030 Olmo di Martellago VE (IT); BERTIN, Walter, 31036 Istrana TV (IT)
(74) Representative: Perani & Partners S.p.A.
(86) International application number: PCT/IB2016/056585
(87) International publication number: WO 2017/077459

(56) References cited:
- WO-A1-2013/121452
- WO-A1-2013/171270
- ZIAD DAOUD ET AL: "Pectin shows antibacterial activity against <i>Helicobacter pylori</i>", ADVANCES IN BIOSCIENCE AND BIOTECHNOLOGY, vol. 04, no. 02, 1 January 2013 (2013-01-01), pages 273-277, XP055281431, ISSN: 2156-8456, DOI: 10.4236/abb.2013.42A037 cited in the application
- MEHDI ZOBEIRI ET AL: "N-acetyl cysteine as an adjunct to standard anti-Helicobacter pylori eradication regimen in patients with dyspepsia: A prospective randomized, open-label trial", ADVANCED BIOMEDICAL RESEARCH, vol. 3, no. 1, 1 January 2014 (2014-01-01) , page 189, XP055281445, ISSN: 2277-9175, DOI: 10.4103/2277-9175.140403 cited in the application
- Dikmen Dokmeci ET AL: "L-carnitine inhibits ethanol-induced gastric mucosal injury in rats", Pharmacological reports : PR, 1 July 2005 (2005-07-01), page 481, XP055281453, Poland Retrieved from the Internet: URL:http://www.if-pan.krakow.pl/pjp/pdf/20 05/4_481.pdf [retrieved on 2016-06-05] cited in the application

## Description

### FIELD OF THE INVENTION

The present invention relates to an oral composition in the form of gel comprising chitosan, pectin with a high degree of methoxylation, a sugary substance, L-carnitine and N-acetyl-cysteine according to the appended claims, for use as a food supplement or as a medicament.

### PRIOR ART

Gastritis is an acute or chronic inflammation with multifactorial etiology which affects the stomach wall at the gastric mucosa level, with the appearance of pain or discomfort in the upper abdomen.

Hydrochloric acid and pepsin, agents which are physiologically produced within the gastric lumen by the parietal cells, are responsible for the gastric irritation at the base of the inflammatory response mediated by stomach cells.

In physiological conditions, the gastric epithelium is coated with a physical barrier consisting of mucus-polysaccharides, intended to protect the inner gastric wall from the detrimental action of hydrochloric acid and enzymes produced in the digestion step.

Alcohol consumption, cigarette smoking, non-steroidal and steroideal anti-inflammatory drugs and *Helicobacter pylori* infections may harm such a protective mucous layer, thereby exposing the stomach wall to the irritating action of gastric juices and causing a gradual thinning thereof. Continuous exposure to said irritating agents can lead, depending on the duration, to a phenomenon of chronic inflammation of the gastric wall with complications more serious than heartburn alone, such as bleeding and epithelial ulceration.

Conditions of increased gastric secretion, in conjunction with anatomical changes of the gastric containment valves, can also induce the onset of associated diseases such as gastric acid reflux, i.e. a phenomenon which involves the abnormal rise of the contents of the stomach to the esophagus.

This organ is not provided with protective systems against the harmful action of hydrochloric acid and is therefore subject to inflammation phenomena similar to those affecting the stomach, with symptoms characterized by a burning feeling in retrosternal position and pain on swallowing, retching and acid belching.

The primary goals of medical therapy of gastritis and disorders associated with it are full control of the symptoms, basically represented by heartburn with or without gastroesophageal reflux, accompanied by improved quality of life and, most importantly, the full restoration of the integrity of the mucosal epithelium and eradicating Helicobacter pylori.

The most common and widespread remedies for the treatment of peptic symptoms are the use of antacids drugs, which include magnesium and aluminum salts, agents neutralizing the excessive acidity of gastric juices, able to limit the erosive action of hydrochloric acid, and the use of "proton pump inhibitors" molecules such as Omeprazole and the newer derivatives Pantoprazole and Lansoprazole, able to interact at a pharmacodynamic level with the ATP-dependent proton pump located in the gastrocytes and responsible for acid hyperproduction.

The compositions containing aluminum and magnesium hydroxides should be administered at a distance of at least two hours after administration of other pharmaceutical preparations since they can interfere with the absorption of numerous active ingredients.

In addition to the above-mentioned buffer systems, another category of drugs commonly used for the control of gastritis-induced symptomatology is that of gastric mucosa protective agents, i.e. compounds capable of forming a synthetic film on the wall of the stomach through the reabsorption of the hydrochloric acid produced.

Once ingested, sodium alginate sequesters the hydrochloric acid present in the gastric lumen, with a salt shifting mechanism, thus forming a gelled structure with barrier properties which is stratified above the gastric secretions and the chyme, thereby mechanically containing the reflux towards the esophagus.

Due to the intrinsic mechanism of action, sodium alginate is mostly used in reflux diseases and is less effective in protecting and healing gastric ulcer as a localized epithelial lesion.

Conversely sucralfate, a film-forming agent widely used for the treatment of reflux esophagitis and gastritis, is layered on the esophagus-gastro-duodenal mucosa and in particular establishes a selective binding with the proteins present at the bottom of ulcerous lesions, forming a protective barrier against the peptic hydrochloride aggression.

Said protective agent requires large amounts of acid to be activated and the intake thereof before meals is therefore suggested; once swollen into the gastric lumen, sucralfate remains in place for up to six hours after ingestion, making the co-administration of systemically acting drugs difficult, whose absorption may be impaired.

### SUMMARY OF THE INVENTION

The Applicant has now surprisingly found that it is possible to make liquid and solid oral formulations with protective action of the gastric wall, which in addition to re-absorbing the hydrochloric acid present in the stomach, promote the re-epithelialization of the gastric mucosa and reduce the incidence of Helicobacter pylori relapse.

The present invention relates to an oral composition in the form of gel comprising chitosan, pectin with a high degree of methoxylation, L-carnitine hydrochloride and/or a pharmaceutically acceptable salt thereof, N-acetylcysteine, a sugary substance or at least one polyol in combination with suitable excipients or diluents, wherein:
i) said gel is an oral suspension characterized by viscosity values of between 1000 and 5000 cPas measured by digital Brookfield viscosity viscometer model Haake Viscotester 6 plus at 20°C;
ii) said chitosan forms a mixed salt with L-carnitine hydrochloride and N-acetylcysteine, wherein the weight ratio of chitosan: L-carnitine hydrochloride: N-acetylcysteine is equal to 0.2: 1:1, with a pH comprised between 3.5 and 4.8 of the composition in liquid form.

The present invention further relates to the above oral composition for use as a supplement or as a medicament in the treatment and prevention of gastritis, gastric reflux esophagitis, peptic ulcer lesions and dystrophic lesions of the oral and digestive mucosa membranes with iatrogenic and radiotherapy basis.

### DESCRIPTION OF THE FIGURES

Figure 1: Formation of a soluble chitosan polycation.
   The Figure provides a graphical representation of the polymer polysalification mechanism in the presence of carnitine hydrochloride and N-acetylcysteine. Chitosan is shown for simplicity as consisting of the D-Glucosamine unit alone, i.e. the unit involved in the formation of the mixed salt.
Figure 2: Mechanism of release of L-carnitine hydrochloride and N-acetylcysteine in the gastric lumen, following the interaction of the soluble chitosan polycation with the hydrochloric acid secreted by the stomach.

### DETAILED DESCRIPTION OF THE INVENTION

Chitosan is a linear polysaccharide consisting of repetitive units of D-glucosamine and N-acetyl-D-glucosamine, bound by β 1-4 glycosidic bonds, whose versatility of use is of great interest from a pharmaceutical point of view.

In fact, the use of chitosan for making mucoadhesive formulations for oral, nasal and ophthalmic application is known, wherein the chemical and mechanical interaction of the polymer with the local mucosa allows a more effective absorption of the drug.

Its gelling and biocompatibility properties are used both in the pharmaceutical field for making modified release formulations and systems, and in other service sectors such as the production of filters for water purification, production of biodegradable films and alternative textile fibers. Pectin is a natural polymer present in the cell wall of all higher plants, consisting of D-galacturonic acid units bound in a linear chain by α 1-4 glycosidic bonds.

Its gelling, thickening and stabilizing properties make it particularly useful for the production of food and cosmetic products, as well as in the pharmaceutical field for the delivery of drugs in the form of matrices, soft capsules and filmed pharmaceutical forms.

Methoxylated pectins are a derivatized form of the polymer, wherein the carboxylic function of D-galacturonic acid is in methoxylated form; more in particular, pectins have a high degree of methoxylation when said functionalization affects at least 50% of the carboxyl groups of the polymer.

For the purposes of the present invention, the pectins used in the preparation of the composition described above are pectins with a high degree of methoxylation.

L-carnitine is a beta hydroxy acid synthesized starting from the two amino acids L-methionine and L-lysine, characterized by a quaternary ammonium group bearing a positive charge.

Said L-carnitine is naturally found in animal foods such as meat and milk, and is used in the nutraceutical industry as a supplement to promote β-oxidation of fatty acids into the mitochondria and thus to produce energy.

N-acetylcysteine (NAC) is a modified amino acid, acetylated derivative of L-cysteine at the amino group; NAC has a dermal and epithelium-trophic activity, ROS (Reactive Oxygen Species) removal activity and mainly mucolytic activity, promoting cleavage of the S-S bond of mucopolysaccharides, for which it is used in the preparation of supplements of pharmaceutical products.

Despite the multiple uses in the pharmaceutical, cosmetic and food industries, the components mentioned above are not used in combination with each other for making appropriate formulations for use as dietary supplements and as medicaments.

The Applicant has now surprisingly found that an oral composition can be made in gel form, comprising the above compounds, wherein the same are able to combine with each other in a specific chemical mechanism, responsible for both the technical and rheological properties of the finished product, crucial for achieving the above actions and for their subsequent release in vivo mediated by contact with the hydrochloric acid in the stomach.

More precisely, said components are used for the formulation of dietary supplements or medicaments for the treatment and prevention of gastritis, gastric reflux esophagitis, peptic ulcer lesions and dystrophic lesions of the oral and digestive mucosa with iatrogenic and radiotherapy basis.

The oral composition in gel form, object of the present patent application, comprises chitosan, pectin with a high degree of methoxylation, a sugary or polyol substance, L-carnitine and/or a pharmaceutically acceptable salt thereof and N-acetylcysteine, in combination with suitable excipients or diluents.

The composition described in the present patent application is in the form of low-viscosity gel, where low-viscosity gel is an oral suspension characterized by viscosity values of between 1000 cPas and 5000 cPas measured by digital Brookfield viscometer model HAAKE Viscotester 6 plus at 20 °C
For the purposes of the present invention, pharmaceutically acceptable salt is a salt suitable for medical use that does not induce toxicity, irritation or allergic reactions when in contact with human and animal living tissues and which has, in terms of biological activity, a reasonable risk/benefit ratio.

Within the scope of the present invention, the pharmaceutically acceptable salt of L-carnitine is hydrochloride.

L-carnitine hydrochloride salt means the ionic compound characterized by the interaction between the quaternary ammonium group of beta-hydroxy acid and the hydrochloric acid chloride ion.

Within the scope of the present invention, sugary substance means a pure organic compound or a complex mixture, characterized by having a sweetening power.

When it is a complex mixture, the sugary substance of the subject composition is preferably honey.

When used as pure, that sugary substance is preferably selected from the compounds belonging to the classes of monosaccharides, where the preferred monosaccharides are dextrose, fructose and mixtures thereof.

Polyols are organic compounds containing at least 2 hydroxyl groups, generally obtained synthetically by sugar reduction.

Preferred are, for example, sorbitol, mannitol, isomalt, inositol; particularly preferred is sorbitol since it is transformed in the human organism into monosaccharides (mainly fructose), without the intervention of insulin; it can therefore be present in the diet of diabetics.

The characteristic chemical mechanism of the subject composition takes place in aqueous environment and involves the formation of a mixed chitosan salt, whose pH in aqueous solution is essential for the delivery of active ingredients as well as the release of the same once the gastric lumen is reached.

In the presence of L-carnitine hydrochloride and NAC, both substances with acid hydrolysis, the pH of the composition in liquid form takes values of between 3.5 and 4.8, by virtue of the acid behavior of the beta-hydroxyacid and of the modified NAC amino acid in aqueous solution.

The chemical environment thus generated (pH of between 3.5 and 4.8) allows the simultaneous formation of a mixed chitosan salt, in which the amino groups of the monomer units of D-glucosamine form ionic interactions with the carboxyl groups of L-carnitine hydrochloride and NAC, thereby generating a polycation.

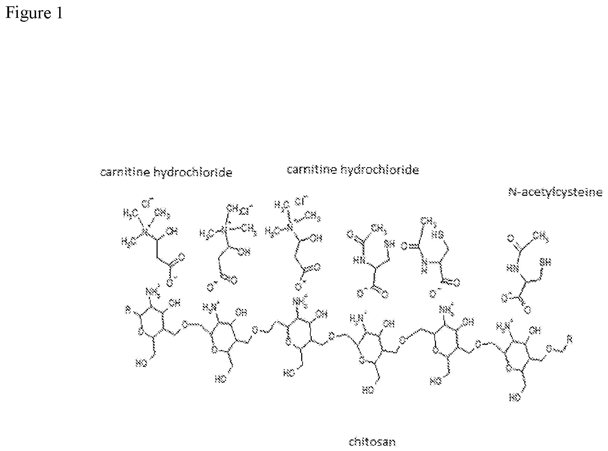

For the purposes of the present invention, mixed salt is a salt in which a single polyanion or polycation interacts with two or more counterions having a different chemical nature at the same time; in this case, the polycation chitosan interacts with L-carnitine and NAC carboxylate anions, thereby forming the respective mixed salt (Figure 1).

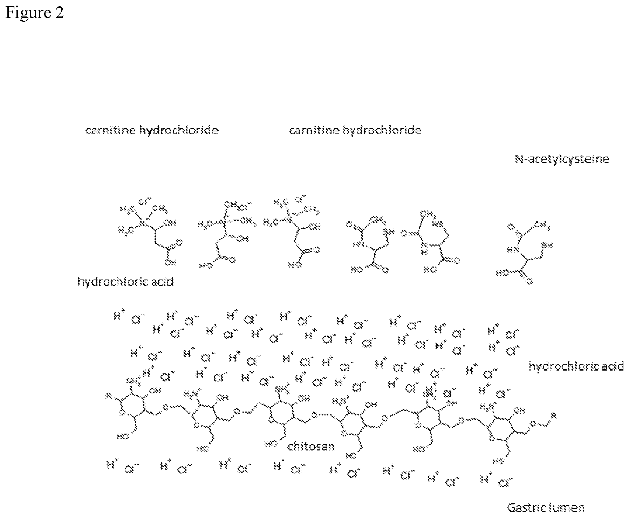

The pH of the composition in liquid form is critical for making an effective formulation which is active at the level of the stomach, i.e. that releases the active ingredients chitosan, L-carnitine hydrochloride and N-acetyl-cysteine once in contact with the gastric juices (Figure 2) and which allows the correct gelling on site of the pectin with a high degree of methoxylation.

It is therefore essential that the pH of the subject composition in liquid form is in the range of between 3.5 and 4.8.

When the above composition reaches the stomach, the totally dissociated hydrochloric acid moves the organic counterions L-carnitine hydrochloride and N-acetyl-cysteine from the amino groups of Chitosan D-glucosamine, allowing the release of the active ingredients contained in the salt which can elicit their mechanisms of action at the gastric mucosa level; at the same time, Chitosan helps subtracting protons from the stomach, thus promoting the increase of the gastric pH.

In the oral composition in gel form of the present invention, the weight ratio of chitosan: L-carnitine hydrochloride: N-acetylcysteine is equal to 0.2: 1:1.

For the purposes of the present invention, the oral composition in gel form has a chitosan content of between 0.1% and 0.3% and a pectin content of between 0.6% and 2.6% by weight on the total weight of the composition.

For the purposes of the present invention, the chitosan used in the preparation of the above composition is preferably vegetable chitosan with a deacetylation degree equal to or greater than 85%.

While chitosan is known for its gelling properties, the oral composition in gel form of the present application contains the polymer in the form of salt and thus in already hydrated form, imparting a gel consistency to the finished formulation.

In contact with gastric fluids, chitosan only swells in a limited manner, because in salt form and already hydrated; however, the polymer is capable of sequestering hydrogen ions from the contents of the stomach, still exhibiting a buffer function.

In this sense, the pectin with a high degree of methoxylation is necessary to obtain a composition in gel form having the right viscosity to stratify along the esophagus and at the bottom of the stomach, where it covers the inflamed mucosa area and dilutes hydrochloric acid.

The mechanism of action whereby pectins with a high degree of methoxylation dilute hydrochloric acid consists in the ability of the functionalized polymer to bind protons by modifying its steric conformation in the aqueous medium and increasing the ability to bind water, thereby swelling.

In other words, in acid environment and in the presence of sugary substances, pectins exert a strong gelling and mechanical barrier power which opposes the gastro-oesophageal reflux; at the same time, they subtract protons to the gastric environment, thus balancing the pH of the stomach.

The dilution of the gel with acidic secretions produces an increase in the local pH, which can increase up to three units compared to the initial conditions (the gastric pH ranges between 1.5 and 2.5), and an increase in the gel viscosity that creates a mechanical barrier on the surface of the gastric mucosa.

In conjunction with these immediate protection effects, there are the skin restoring, antibacterial effects and prevention of Helicobacter pylori relapses exercised by L-Carnitine, NAC and pectins.

Chitosan is known in literature for its skin restoration and healing properties against injured mucosa; in particular, said polymer seems to prevent the reduction of ethanol-induced mucus in rats gastric mucosa (Mikio Ito, Ayako Ban, Masahi Ishihara. Anti-Ulcer Effects of Chitin and Chitosan, Healthy Foods, In Rats. Japanese Journal of Pharmacology, 82, 218-225 (2000)). Compositions comprising chitosan are also known for the treatment of gastroesophageal reflux from WO 2013/121452 A1, wherein chitosan is reported to modulate the acidity rate of the gastric content, thus preventing it to rise into the esophagus.

A scientific study conducted by Z. Daoud in October 2012 showed that pectin exhibits a particularly effective antibacterial activity at acid pH against infection by Helicobacter pylori (Ziad Daoud, Mihir Sura, Roula m., Abdel-Massih. Pectin shows antibacterial activity against Helicobacter pylori. Advances in Biosciences and Biotechnology, 2013, 4, 273-277).

The ingestion of N-acetyl-cysteine appears to be related to lower risk of relapse of ulcer by Helicobacter pylori, also in combination with antibiotic therapy; it is believed that said amino acid promotes the re-epithelialization of the mucous membranes through an oxidative stress reduction mechanism characteristic of tissue degeneration (Mohammad Hassan Enami, Mehdi Zobeiri, Hojatolah Rahimi, Fariba Arjomandi, Hamed Daghagzadeh, Pyman Adibi, Jalal Hashemi. N-acetyl cysteine as an adjunct to standard anti-Helicobacter pylori eradication regimen in patients with dyspepsia: A prospective randomized, open-label trial. Advanced Biomedical Research, 2014 Sep, 8; 3: 189; Makipour K, Friedenberg FK. The Potential Role Of N-Acetylcysteine For The Treatment Of Helicobacter Pylori. Journal of Clinical Gastroenterology, 2011; 45(10): 841-843; Gurbuz AK1, Ozel AM, Ozturk R, Yildirim S, Yazgan Y, Demirturk L. Effect Of N-Acetyl Cysteine On Helicobacter Pylori. South Medical Journal, 2005 Nov; 98(11):1095-7).

Scientific studies conducted on rats have allowed the identification of an in vivo correlation between the intake of L-carnitine and gastro-protective effect against gastritis induced by non-steroidal anti-inflammatory drugs and alcohol (Dikmen Dokmeci, Meryem Akpolat, Nurettin Aydogdu, Latife Doganay, F. Nesrin Turan. L-carnitine inhibits ethanol-induced gastric mucosal injury in rats. Pharmacological Reports 2005, 57, 481-488,; Izgut-Uysal VN, Agac A, Derin N. Effect of carnitine on stress-induced lipid peroxidation in rat gastric mucosa. Journal of Gastroenterology, 2001 Apr, 36(4): 231-6; Derin N, Izgut-Uysal VN, Agac A, Aliciguzel Y, Demir N. L-carnitine protects gastric mucosa by decreasing ischemia-reperfusion induced lipid peroxidation. Journal of physiology and pharmacology: an official journal of the Polish Physiological Society 2004 Sep; 55(3): 595-606).

The oral composition in gel form described in the present invention allows the delivery of all the above components in a single formulation, through the formation of the mixed chitosan salt, wherein the specific pH of between 3.5 and 4.8 is functional on the one hand to the formation of a protective mechanical barrier, on the other hand to the release of the active ingredients in vivo for the therapeutic effect.

The partial hydration of formulation has the great advantage of not requiring large amounts of juices to be activated and to dilute promptly in the gastric secretions already at first contact with the same, immediately and efficiently reducing the gastric hyperacidity perceived by the patient, without the administration and interaction drawbacks related to sucralfate and sodium alginate and overcoming the limits of efficacy already described above.

For the purposes of the present application, the subject oral composition in gel form is intended for human and animal use in the form of a dietary supplement or medicament.

The oral composition of the present invention is in gel form upon ingestion and can be formulated in either solid or liquid pharmaceutical forms, wherein said solid pharmaceutical forms are to be reconstituted in water prior to administration.

More preferably, the solid pharmaceutical forms to be reconstituted in water are, in the present patent application, tablets, capsules and powders.

More preferably, the liquid pharmaceutical forms in which the oral composition of the present invention may be formulated are gels and syrups, provided that said final pharmaceutical form maintains a viscosity of between 1000cPas and 5000cPas that allows the layering of the composition in vivo.

In particular, the oral composition in gel form of the present invention is intended for the treatment and prevention of gastritis, gastric reflux esophagitis, peptic ulcer lesions and dystrophic lesions of the oral and digestive mucosa with iatrogenic and radiotherapy basis. Dystrophic lesions of oral and digestive mucosa on iatrogenic and radiotherapy basis are the anatomical and functional alterations of the same, related to the continuous use of drugs and/or medical therapies and in particular to the toxicity inherent in the treatment itself.

Very common iatrogenic diseases are those produced by the chronic use of steroideal and non-steroidal anti-inflammatory drugs that are necessary to control acute inflammation or most often very serious arthritis and osteoarthritis.

Treatment with antitumor radio- and chemotherapy is often responsible for iatrogenic lesions in the oral mucosa, such as stomatitis and mucositis.

The oral composition in gel form of the present invention can in fact also be used for the treatment and prevention of esophageal lesions induced by acid reflux and for stomatitis and mucositis, as phenomena caused by the decrease of the defenses normally present in the oral cavity or of the systemic immunity defenses and by the antitumor radio-chemotherapy.

Factors that may contribute to the onset of gastritis, esophagitis, gastric ulcer lesions and mucosal dystrophic lesions of the oral and digestive tract may be multiple and include improper eating habits, stress, cigarette smoking, alcohol consumption, consumption of acidic beverages, congenital abnormalities of the gastric valves that may lead to gastric reflux, habitual use of non-steroideal anti-inflammatory drugs (NSAIDs), chemo- and radiotherapy (mucositis).

For the purposes of the present invention, the oral composition in gel form claimed may be also used for the treatment and prevention of gastritis induced by bacterial infection of Helicobacter pylori.

The oral composition in gel form which is the subject of the present patent application preferably comprises honey as dispersing agent of the active ingredients and promoting the gelling of pectins; said substance, for its sweetening, soothing and regenerating power, can be of particular help for masking the flavor of the composition in addition to having itself a bactericidal and regenerating action.

The viscosity of honey and its hydrophilicity also allow mechanically controlling the gastric acid reflux and at the same time diluting the acidic juices from the stomach.

In this case, the oral composition is preferably prepared with a process that comprises the following steps:
**a.** Dispersing pectins in honey under stirring to obtain a homogeneous system.
**b.** Adding purified water at successive volumes to the mixture obtained from **a.,** leaving the system under stirring until complete homogenization of the components.
**c.** Adding L-carnitine and/or its pharmaceutically acceptable salt, a preservative and N-acetyl-cysteine to the mixture obtained from **b.,** leaving under stirring until complete dispersion.
**d.** Disperse chitosan in the mixture obtained from **c.** and let hydrate.
**e.** Add the flavor to mixture **d.**
**f.** Check that the pH of mixture **e.** is between 3.8 and 4.5.
**g.** Stir the final mixture obtained from **f.**

For the purposes of the present invention, suitable excipients or diluents are all those components that can make the formulation technologically suitable for its administration.

More preferably, said excipients or diluents are: purified water, flavorings, preservatives; more precisely, the preservative preferably used in the subject composition of the present invention is potassium sorbate.

### EXAMPLE

An example of the preparation of an oral formulation of in gel, according to the purposes of the present invention, is given by way of non-limiting illustration.

| **Component** | **Concentration weight/total composition weight** |
|---|---|
| Vegetable min. chitosan 85% | 0.20% |
| Pectin with a high degree of methoxylation | 1.60% |
| L-Carnitine base | 4.00% |
| L-carnitine hydrochloride | 1.00% |
| Flower honey | 45.00% |
| Purified water | 46.80% |
| Potassium sorbate | 0.10% |
| Liquid all fruits flavor | 0.30% |
| N-acetyl-L-cysteine | 1.00% |

The oral composition in gel form thus formed is prepared according to the process described hereinafter (per 100 grams of product):
**a.** Dispersing pectins in honey under stirring to obtain a homogeneous system.
**b.** Adding purified water 5 g at a time to the mixture obtained from **a.,** leaving the system under stirring until complete homogenization of the components.
**c.** Adding L-carnitine and/or its pharmaceutically acceptable salt, a preservative and N-acetyl-cysteine to the mixture obtained from **b.,** leaving under stirring until complete dispersion.
**d.** Disperse chitosan in the mixture obtained from **c.** and let hydrate.
**e.** Add the flavor to mixture **d.**
**f.** Check that the pH of mixture **e.** is between 3.8 and 4.5.
**g.** Stir the final mixture obtained from **f.**

## Claims

1. Oral composition in gel form comprising chitosan, pectin with a high degree of methoxylation, L-carnitine hydrochloride and/or a pharmaceutically acceptable salt thereof, N-acetylcysteine, a sugary substance or at least one polyol in combination with suitable excipients or diluents, wherein
i) said gel is an oral suspension **characterized by** viscosity values of between 1000 and 5000cPas measured by digital Brookfield viscosity viscometer model Haake Viscotester 6 plus at 20°C;
ii) said chitosan forms a mixed salt with L-carnitine hydrochloride and N-acetylcysteine wherein the weight ratio of chitosan: L-carnitine hydrochloride: N-acetylcysteine is equal to 0.2: 1:1, with a pH comprised between 3.5 and 4.8 of the composition in liquid form

2. Oral composition in gel form according to claim 1, wherein the content of chitosan is comprised between 0.1 % and 0.3 % by weight on the total weight of the composition.

3. Oral composition in gel form according to any one of claims 1 or 2, wherein the content of pectin with a high degree of methoxylation is comprised between 0.6 % and 2.6 % by weight on the total weight of the composition.

4. Oral composition in gel form according to any one of claims from 1 to 3, wherein the sugary substance is honey.

5. Oral composition in gel form according to any one of claims from 1 to 4, wherein the polyol is sorbitol.

6. Oral composition in gel form according to any one of claims from 1 to 5, for use in the treatment and prevention of at least one disease selected in the group consisting of gastritis, gastric reflux esophagitis, peptic ulcer lesions and dystrophic lesions of the oral and digestive mucosa with iatrogenic and radiotherapy basis.

7. Oral composition in gel form for use according to claim 6, wherein said gastritis and peptic ulcer lesions are induced by Helycobacter pylori.

8. Oral composition in gel form according to any one of claims from 1 to 5, for human or animal use as a dietary supplement or a medicament.

9. Oral compositions in gel form according to anyone of claims 1-5, wherein, they are solid forms to be reconstituted in water prior to administration, or liquid forms of gel or syrup, provided that the final pharmaceutical form is a gel having the viscosity value according to claim 1.

10. Oral composition according to claim 9, wherein said solid forms to be reconstituted in water are selected from tablets, capsules and powders.

## Patentansprüche

1. Orale Zusammensetzung in Gelform, umfassend Chitosan, Pektin mit einem hohen Methoxylierungsgrad, L-Carnitinhydrochlorid und/oder ein pharmazeutisch verträgliches Salz davon, N-Acetylcystein, eine zuckerhaltige Substanz oder mindestens ein Polyol in Kombination mit geeigneten Trägerstoffen oder Verdünnungsmitteln, wobei
i) das Gel eine orale Suspension ist, **gekennzeichnet durch** Viskositätswerte zwischen 1000 und 5000 cPas, gemessen mit dem digitalen Brookfield-Viskositätsviskosimeter Modell Haake Viscotester 6 plus bei 20°C;
ii) das Chitosan ein gemischtes Salz mit L-Carnitinhydrochlorid und N-Acetylcystein bildet, wobei das Gewichtsverhältnis von Chitosan: L-Carnitinhydrochlorid: N-Acetylcystein 0,2 betragt: 1:1, mit einem pH-Wert zwischen 3,5 und 4,8 der Zusammensetzung in flüssiger Form.

2. Orale Zusammensetzung in Gelform nach Anspruch 1, wobei der Gehalt an Chitosan zwischen 0,1 Gew.- % und 0,3 Gew.- % liegt, bezogen auf das Gesamtgewicht der Zusammensetzung.

3. Orale Zusammensetzung in Gelform nach einem der Ansprüche 1 oder 2, wobei der Gehalt an Pektin mit einem hohen Methoxylierungsgrad zwischen 0,6 und 2,6 Gew.- % liegt, bezogen auf das Gesamtgewicht der Zusammensetzung.

4. Orale Zusammensetzung in Gelform nach einem der Ansprüche 1 bis 3, wobei die zuckerhaltige Substanz Honig ist.

5. Orale Zusammensetzung in Gelform nach einem der Ansprüche 1 bis 4, wobei das Polyol Sorbitol ist.

6. Orale Zusammensetzung in Gelform nach einem der Ansprüche 1 bis 5 zur Verwendung bei der Behandlung und Prävention auf iatrogener und Strahlentherapiebasis von mindestens einer Erkrankung, ausgewählt aus der Gruppe bestehend aus Gastritis, gastrischer Refluxösophagitis, peptischen Ulkusläsionen und dystrophischen Läsionen der Mund- und Verdauungsschleimhaut.

7. Orale Zusammensetzung in Gelform zur Verwendung nach Anspruch 6, wobei die Gastritis- und peptischen Ulkusläsionen durch Helycobacter pylori induziert werden.

8. Orale Zusammensetzung in Gelform nach einem der Ansprüche 1 bis 5 zur menschlichen oder tierischen Verwendung als Nahrungsergänzungsmittel oder Medikament.

9. Orale Zusammensetzungen in Gelform nach einem der Ansprüche 1 bis 5, wobei es sich um feste Formen handelt, die vor der Verabreichung in Wasser rekonstituiert werden sollen, oder um flüssige Formen von Gel oder Sirup, mit der Maßgabe, dass die endgültige pharmazeutische Form ein Gel mit dem Viskositätswert nach Anspruch 1 ist.

10. Orale Zusammensetzung nach Anspruch 9, wobei die festen Formen, die in Wasser rekonstituiert werden sollen, aus Tabletten, Kapseln und Pulvern ausgewählt sind.

## Revendications

1. Composition orale sous forme de gel comprenant du chitosane, de la pectine à haut degré de méthoxylation, du chlorhydrate de L-carnitine et/ou un sel pharmaceutiquement acceptable de celui-ci, de la N-acétylcystéine, une substance sucrée ou au moins un polyol en combinaison avec des excipients ou diluants appropriés, où
i) ledit gel est une suspension buvable **caractérisée par** des valeurs de viscosité comprises entre 1000 et 5000cPas mesurées par viscosimètre numérique Brookfield modèle Haake Viscotester 6 plus à 20°C ;
ii) ledit chitosane forme un sel mélangé avec du chlorhydrate de L-carnitine et de la N-acétylcystéine, où le rapport pondéral du chitosane : chlorhydrate de L-carnitine : N-acétylcystéine est égal à 0,2: 1:1, avec un pH compris entre 3,5 et 4,8 de la composition sous forme liquide.

2. Composition orale sous forme de gel selon la revendication 1, où la teneur en chitosane est comprise entre 0,1 % et 0,3 % en poids par rapport au poids total de la composition.

3. Composition orale sous forme de gel selon l'une quelconque des revendications 1 ou 2, où la teneur en pectine à haut degré de méthoxylation est comprise entre 0,6 % et 2,6 % en poids par rapport au poids total de la composition.

4. Composition orale sous forme de gel selon l'une quelconque des revendications 1 à 3, où la substance sucrée est du miel.

5. Composition orale sous forme de gel selon l'une quelconque des revendications 1 à 4, où le polyol est du sorbitol.

6. Composition orale sous forme de gel selon l'une quelconque des revendications 1 à 5, destinée à être utilisée dans le traitement et la prévention d'au moins une maladie choisie dans le groupe constitué par la gastrite, l'œsophagite par reflux gastrique, les lésions ulcéreuses peptiques et les lésions dystrophiques de la muqueuse buccale et digestive à base iatrogène et radiothérapeutique.

7. Composition orale sous forme de gel destinée à être utilisée selon la revendication 6, où ladite gastrite et lesdites lésions d'ulcère peptique sont induites par Helicobacter pylori.

8. Composition orale sous forme de gel selon l'une quelconque des revendications 1 à 5, pour une utilisation humaine ou animale en tant que complément alimentaire ou médicament.

9. Compositions orales sous forme de gel selon l'une quelconque des revendications 1 à 5, où il s'agit de formes solides à reconstituer dans l'eau avant administration, ou de formes liquides de gel ou de sirop, à condition que la forme pharmaceutique finale soit un gel ayant la valeur de viscosité selon la revendication 1.

10. Composition orale selon la revendication 9, où lesdites formes solides à reconstituer dans l'eau sont choisies parmi les comprimés, les capsules et les poudres.
